(19) Europäisches Patentamt / European Patent Office / Office européen des brevets

(11) **EP 3 460 500 A1**

(12) **EUROPÄISCHE PATENTANMELDUNG**

(43) Veröffentlichungstag:
**27.03.2019 Patentblatt 2019/13**

(51) Int Cl.:
**G01R 33/3873** (2006.01)   **G01R 33/48** (2006.01)
**G01R 33/3875** (2006.01)

(21) Anmeldenummer: **17193198.3**

(22) Anmeldetag: **26.09.2017**

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**MA MD**

(71) Anmelder: **Siemens Healthcare GmbH**
**91052 Erlangen (DE)**

(72) Erfinder: **Leghissa, Martino**
**91369 Wiesenthau (DE)**

(54) **MEDIZINISCHES BILDGEBUNGSGERÄT ZUR KOMBINIERTEN MAGNETRESONANZBILDGEBUNG UND BESTRAHLUNG UND VERFAHREN ZUR BESTIMMUNG DER BESTÜCKUNG VON SHIM-EINHEITEN**

(57) Medizinisches Bildgebungssystem zur kombinierten Magnetresonanzbildgebung und Bestrahlung eines Untersuchungsobjektes, aufweisend eine Magnetresonanzbildgebungseinheit und eine Strahlungserzeugungseinheit, welche mechanisch miteinander derart verbunden sind, dass die Strahlungserzeugungseinheit mit der Magnetresonanzbildgebungseinheit baulich integriert ist und beide eine Patientenöffnung zur Lagerung des Untersuchungsobjektes umgeben, wobei die Magnetresonanzbildgebungseinheit zumindest einen Hauptmagneten zur Erzeugung eines Magnetfelds aufweist und wobei die Strahlungserzeugungseinheit eine Strahlenquelle zur Erzeugung einer Strahlung aufweist, wobei das Bildgebungssystem aus einem statischen Teil und einem um einen Rotationswinkel rotierbaren Teil derart ausgebildet ist, dass entweder zumindest ein drehbarer Hauptmagnet um eine statische Strahlungserzeugungseinheit rotierbar angeordnet ist oder eine drehbare Strahlungserzeugungseinheit um zumindest einen statischen Hauptmagneten rotierbar angeordnet ist, wobei das Bildgebungssystem zumindest zwei passive Shim-Einheiten aufweist, von denen zumindest eine statische Shim-Einheit feststehend ist und zumindest eine drehbare Shim-Einheit mit dem rotierbaren Teil fest verbunden ist.

FIG 1

**Beschreibung**

Die Erfindung betrifft ein Medizinisches Bildgebungssystem zur kombinierten Magnetresonanzbildgebung und Bestrahlung eines Untersuchungsobjektes gemäß dem Patentanspruch 1 sowie ein Verfahren zur Bestimmung der Bestückung von Shim-Einheiten mit Shimelementen gemäß dem Patentanspruch 7.

Wird ein Magnetresonanztomographiesystem in Kombination mit einer integrierten Strahlenquelle betrieben, also zum Beispiel mit einer Röntgenquelle oder einer LINAC (linear particle accelerator; Strahlentherapiequelle), dann wird aus klinischen Gründen in den meisten Fällen eine Einstellung des Applikationswinkels der Strahlung relativ zum Patient gewünscht. Dazu rotiert entweder (a) der MR Magnet um seine longitudinale Achse oder (b) die übrigen Komponenten um den statischen MR Magneten. Die integrierte Strahlenquelle muss zudem relativ zum Untersuchungsobjekt verschiedene Angulationen einnehmen können.

Bei der Magnetresonanzbildgebung ist ein möglichst homogenes Magnetfeld $B_0$ notwendig. Die notwendige Feldhomogenität befindet sich im Allgemeinen in der Größenordnung von wenigen ppm und niedriger. Technisch ist es nicht möglich, Magneten mit perfekter Feldhomogenität zu erzeugen, vor allem durch Einschränkungen bei der Herstellung, eine große Anzahl von Variablen beim Herstellungsprozess und mechanische und elektrische Toleranzen. Zusätzlich beeinflussen umgebende Strukturen das Magnetfeld und erzeugen Feldverzerrungen.

In der Magnettechnologie wird zum Ausgleich kleiner Inhomogenitäten, die üblicherweise in Magnetfeldern vorhanden sind, das sogenannte shimmen (shimming) verwendet, wobei zwischen aktivem und passivem shimmen unterschieden wird. Für das passive shimmen wird im Allgemeinen während der Installation des Magneten magnetisiertes Material an bestimmten Stellen des MR-Scanners angeordnet. Für das aktive shimmen werden speziell hergestellte stromdurchflossene Spulen (ähnlich Gradientenspulen) verwendet. Der Strom kann entsprechend verändert werden, um die Homogenität des Magnetfeldes feineinzustellen.

Durch das Shimmen wird die Feldhomogenität innerhalb des darzustellenden Volumens entsprechend der gewünschten Qualität verbessert. Beim passiven Shimmen werden dabei ferromagnetische Materialien, typischerweise Eisen oder Stahl, in regulären Mustern an speziellen Stellen entlang der inneren Öffnung des Magneten verteilt angeordnet. Eine übliche Anordnung für zylindrische Supraleitungsscanner beinhaltet zwischen 12 und 24 Trägerschubladen, sogenannte "trays", die symmetrisch um den Umfang des Magneten verteilt sind. Jede Trägerschublade ist entlang der z-Achse des Scanners in einer Art Kanal aufgenommen und enthält Fächer, in welche eine gewünschte Anzahl von ferromagnetischen Shimelementen eingelegt werden können.

Bekannte MR Bildgebungssysteme nutzen im Allgemeinen gleichzeitig passives und aktives Shimmen. Aktives Shimmen wird nur verwendet, um Feldverzerrungsharmonien niedriger Ordnung abzuschirmen, typischerweise erster und zweiter Ordnung. Höhere Ordnungen werden durch passives Shimmen unterdrückt. Der Vorteil des aktiven Shimmens liegt darin, dass eine dynamische Anpassung der durch die Spulen fließenden Ströme vorgenommen werden kann. Dadurch kann eine Anpassung an das jeweilige Untersuchungsobjekt erfolgen. In modernen MR Systemen wird in der Vorbereitung vor der Untersuchung routinemäßig schnelles automatisiertes Shimmen durchgeführt. Der Nachteil des passiven Shimmens liegt darin, dass es sich um eine statische Lösung handelt. Wenn sicher der Magnet verändert oder ausgetauscht wird oder wenn sich die Umgebung des Magneten ändert, wird das Magnetfeld typischerweise inhomogener. So muss auch das Shimmen angepasst werden, wenn der Magnet um seine longitudinale Achse um seine Umgebung rotiert oder wenn die Umgebung um den statischen Magneten rotiert.

In dem Artikel "From static to dynamic 1.5T MRI-linac prototype: impact of gantry position related magnetic field variation on image fidelity", von Sjoerd Crijns und Bas Raaymakers, Phys. Med. Biol. 59, Seiten 3241-3247, 2014, werden magnetische Feldvariationen eines Partikeltherapie-LINAC, welches auf einer Gantry um einen festen MR-Magneten rotiert, untersucht. Feldinhomogenitäten werden dabei reduziert, indem die Spulen für das aktive Shimmen winkelabhängige angesteuert werden. Es werden ausschließlich aktive Shims erster Ordnung (Linergradient) verwendet, Inhomogenitäten höherer Ordnung werden nicht angesprochen.

In dem Artikel "Geometric distortion and shimming considerations in a rotating MR-linac design due to the influence of low-level externa magnetic fields" von K. Wachowicz, T. Tadic und B.G. Fallone, Med. Phys. 39(5), Seiten 2659-2668, 2012, werden theoretische Lösungen für das Shimmen bei einem rotierenden MR-LINAC-System diskutiert. Die beschrieben Lösung erwähnt eine passive Shimeinheit, die mit dem rotierenden Magneten verbunden ist und zusätzlich eine aktive Shimeinheit für Verzerrungen erster und zweiter Ordnung. Es werden verschiedene Ansätze untersucht, so z.B. eine passive Shimeinheit, die für einen einzigen Rotationswinkel optimiert ist oder eine passive Shimeinheit, die eine mittlere Abdeckung für den gesamten Winkelbereich bietet.

Es ist Aufgabe der vorliegenden Erfindung, ein medizinisches Bildgebungsgerät zur kombinierten Magnetresonanzbildgebung und Bestrahlung eines Untersuchungsobjekts bereitzustellen, bei welchem auch bei teilweise rotierenden Komponenten Magnetfeldverzerrungen insbesondere auch höherer Ordnung möglichst effektiv abgeschirmt werden; des Weiteren ist es Aufgabe der Erfindung, ein Verfahren zur Bestückung von Shimeinheiten zur Kompensation der Magnetfeldverzerrungen bei teilweise rotierenden Komponenten vorzusehen.

Die Aufgabe wird erfindungsgemäß gelöst durch ein Medizinisches Bildgebungssystem zur kombinierten Magnetresonanzbildgebung und Bestrahlung eines Untersuchungsobjektes gemäß dem Patentanspruch 1 und von einem Verfahren zur Bestimmung der Bestückung von Shim-Einheiten mit Shimelementen gemäß dem Patentanspruch 7. Vorteilhafte Ausgestaltungen der Erfindung sind jeweils Gegenstand der zugehörigen Unteransprüche.

Durch das Medizinische Bildgebungssystem zur kombinierten Magnetresonanzbildgebung und Bestrahlung eines Untersuchungsobjektes, aufweisend eine Magnetresonanzbildgebungseinheit und eine Strahlungserzeugungseinheit, welche mechanisch miteinander derart verbunden sind, dass die Strahlungserzeugungseinheit mit der Magnetresonanzbildgebungseinheit baulich integriert ist und beide eine Patientenöffnung zur Lagerung des Untersuchungsobjektes umgeben, wobei die Magnetresonanzbildgebungseinheit zumindest einen Hauptmagneten zur Erzeugung eines Magnetfelds aufweist und wobei die Strahlungserzeugungseinheit eine Strahlenquelle zur Erzeugung einer Strahlung aufweist, wobei das Bildgebungssystem aus einem statischen Teil und einem um einen Rotationswinkel rotierbaren Teil derart ausgebildet ist, dass entweder zumindest ein drehbarer Hauptmagnet um eine statische Strahlungserzeugungseinheit rotierbar angeordnet ist oder eine drehbare Strahlungserzeugungseinheit um zumindest einen statischen Hauptmagneten rotierbar angeordnet ist, wobei das Bildgebungssystem zumindest zwei passive Shim-Einheiten aufweist, von denen zumindest eine statische Shim-Einheit feststehend ist und zumindest eine drehbare Shim-Einheit mit dem rotierbaren Teil fest verbunden ist, ist es möglich, Magnetfeldverzerrungen ganz besonders effektiv zu kompensieren. Jede der zumindest zwei passiven Shim-Einheiten übernimmt dabei einen Anteil der Kompensierung der Magnetfeldverzerrung. Eine geeignete Aufteilung kann somit ein optimales Kompensierungsergebnis erzielen. Die Magnetfeldverzerrungen als solche sind ursprünglich bedingt teilweise durch Einschränkungen bei der Qualität des (Haupt)magneten und teilweise durch andere Bauteile des Bildgebungssystems oder die Umgebung.

Nach einer Ausgestaltung der Erfindung weisen die zumindest zwei passiven Shim-Einheiten jeweils eine Vielzahl von Shimelementen auf und sind die Shimelemente in den Shim-Einheiten derart angeordnet und verteilt, dass sie Magnetfeldverzerrungen zumindest teilweise, insbesondere zu mindestens 95% kompensieren.

Insbesondere sind die zwei passiven Shim-Einheiten dafür ausgebildet, Magnetfeldverzerrungen zumindest zweiter und/oder höherer Ordnung zu kompensieren.

Nach einer Ausgestaltung der Erfindung ist die zumindest eine statische Shim-Einheit derart ausgebildet, dass sie eine erste Komponente der Magnetfeldverzerrungen, welche unabhängig von dem Rotationswinkel ist, zumindest teilweise () kompensiert und ist die zumindest eine drehbare Shim-Einheit derart ausgebildet, dass eine zweite Komponente der Magnetfeldverzerrungen, die abhängig von dem Rotationswinkel ist, zumindest teilweise kompensiert. Eine derartige Aufteilung der zu kompensierenden Magnetfeldverzerrungen in Rotationswinkelabhängige und -unabhängige Magnetfeldverzerrungen und Kompensierung durch die entsprechende Shim-Einheit gewährleistet ein besonders homogenes Magnetfeld $B_0$ als Resultat. Dadurch ist eine qualitativ besonders hochwertige MR-Bildgebung möglich.

Nach einer Ausgestaltung der Erfindung wird die Strahlungserzeugungseinheit von einer Röntgenbildgebungseinheit gebildet, und weist die Röntgenbildgebungseinheit eine Röntgenquelle zur Erzeugung eines Röntgenstrahls auf. Kombinierte Magnetresonanzbildgebungs- und Röntgenbildgebungsgeräte sind besonders geeignet, eine umfassende und sich gegenseitig ergänzende Darstellung von Knochen, Organen und Gewebe zu bieten, welche zur Diagnose der verschiedensten Krankheiten genutzt werden können. Zweckmäßigerweise weist die Röntgenbildgebungseinheit außerdem einen Röntgendetektor zur Umwandlung von Strahlung in Bilddaten auf.

Zur Kompensation weiterer Magnetfeldverzerrungen besonders niedriger Ordnung ist in vorteilhafter Weise zusätzlich zumindest eine aktive Shimeinheit mit einer Vielzahl von Spulen, wobei die Spulen bezüglich ihres Durchflussstroms ansteuerbar ausgebildet sind, vorgesehen.

Die Erfindung umfasst außerdem ein Verfahren zur Bestimmung der Bestückung von Shim-Einheiten mit Shimelementen bei einem oben genannten medizinischen Bildgebungssystem mit den folgenden Schritten: Messen der Feldverteilung des Magnetfeldes der Magnetresonanzbildgebungseinheit, insbesondere innerhalb der Patientenöffnung, abhängig von dem Rotationswinkel und daraus Bestimmung der Magnetfeldverzerrung, Bestimmung einer ersten Komponente der Magnetfeldverzerrung, die unabhängig vom Rotationswinkel ist und einer zweite Komponente, die abhängig vom Rotationswinkel ist, Bestimmung der Verteilung von Shimelementen in der statischen Shim-Einheit zur Kompensierung der ersten Komponente der Magnetfeldverzerrung, und Bestimmung der Verteilung von Shimelementen in der drehbaren Shim-Einheit zur Kompensierung der zweiten Komponente der Magnetfeldverzerrung. Insbesondere wird die Anzahl, Größe und Position der Shimelemente in den Shim-Einheiten bestimmt bzw. berechnet, um anschließend die Bestückung auf einfache Weise durchführen zu können.

Nach einer weiteren Ausgestaltung der Erfindung werden die Shimelemente gemäß der berechneten Verteilung in die Shim-Einheiten eingelegt. Dies kann manuell z.B. durch einen Servicetechniker oder auch automatisch durch eine Bestückungsvorrichtung durchgeführt werden. Anschließend kann durch nochmaliges Messen der Feldverteilung des Magnetfeldes überprüft werden, ob die Kompensierung ausreichend ist, eventuell kann eine weitere Berechnung und ein weiteres Bestücken durchgeführt werden.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:

FIG 1    eine Ansicht eines erfindungsgemäßen Bildgebungssystems mit zwei passiven Shimeinheiten;

FIG 2    eine Ansicht des Bildgebungssystems nach FIG 1 mit um einen Rotationswinkel Θ gedrehtem Hauptmagnet;

FIG 3    ein Ablauf eines Verfahrens zur Bestimmung der Bestückung von Shim-Einheiten;

FIG 4    eine beispielhafte Messung einer Magnetfeldverzerrung in Abhängigkeit vom Rotationswinkel vor dem Shimmen;

FIG 5    eine beispielhafte Messung einer Magnetfeldverzerrung in Abhängigkeit vom Rotationswinkel nach dem Shimmen durch eine passive statische Shimeinheit; und

FIG 6    eine beispielhafte Messung einer Magnetfeldverzerrung in Abhängigkeit vom Rotationswinkel nach dem Shimmen durch eine passive statische Shimeinheit und eine passive rotierende Shimeinheit.

In den folgenden Figuren ist die Erfindung anhand eines medizinischen Bildgebungssystems mit einem rotierenden Hauptmagneten der Magnetresonanzbildgebungseinheit und einer statischen Röntgenbildgebungseinheit beschrieben, es kann jedoch alternativ auch die Magnetresonanzbildgebungseinheit statisch und die Röntgenbildgebungseinheit rotierbar sein.

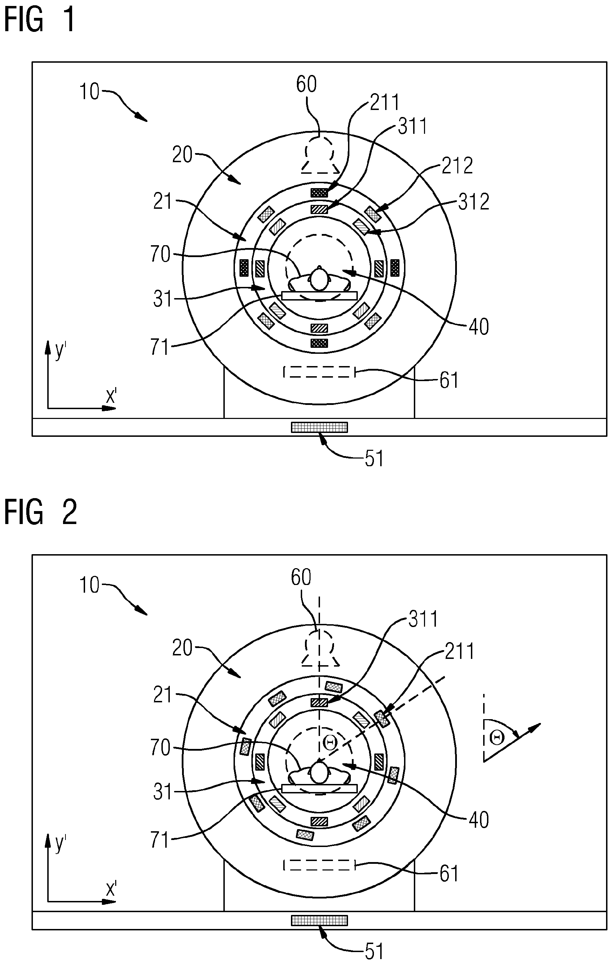

In der FIG 1 ist ein medizinisches Bildgebungssystem 10 gezeigt, welches eine Magnetresonanzbildgebungseinheit mit einem um eine Öffnung 40 rotierenden Hauptmagneten 20 aufweist sowie eine statische Röntgenbildgebungseinheit mit einer Röntgenquelle 60 und einem Röntgendetektor 61. Das Bildgebungssystem 10 weist außerdem eine mit dem rotierbaren Hauptmagneten 20 rotierbare passive Shimeinheit 21 und eine statische passive Shimeinheit 31 auf. Es kann dabei vorgesehen sein, dass die rotierbare passive Shimeinheit 21 mit dem Hauptmagneten 20 baulich derart verbunden ist, dass bei einer Rotation des Hauptmagneten 20 die rotierbare passive Shimeinheit 21 automatisch entsprechend mitrotiert. Die Magnetresonanzbildgebungseinheit und die Röntgenbildgebungseinheit umgeben die Patientenöffnung 40, in welcher wiederum ein Patiententisch 71 mit einem darauf gelagerten Patienten 70, das Untersuchungsobjekt (im Allgemeinen ein Teil/Organ des Patienten) angeordnet ist.

Die beiden passiven Shimeinheiten sind dazu ausgebildet, Magnetfeldverzerrungen des gesamten Magnetfeldes zu kompensieren. Die Magnetfeldverzerrungen kommen teilweise durch Qualitätseinschränkungen des Hauptmagneten, durch magnetische Bauteile des Bildgebungssystems und durch magnetische Elemente 51 der Umgebung des Bildgebungssystems zu Stande. Zur Kompensierung weist jede Shimeinheit mehrere Trägerschubladen mit Taschen auf, welche Taschen wiederum mit einer Vielzahl von Shimelementen, im Allgemeinen ferromagnetische Eisenbleche, bestückbar sind. Die Shimelemente werden derart in den Taschen der Trägerschubladen angeordnet, dass sie die Magnetfeldverzerrungen kompensieren können. Die rotierbare Shimeinheit 21 weist in der Darstellung eine erste Trägerschublade 211 und eine zweite Trägerschublade 212 sowie sechs weitere Trägerschubladen auf, die statische Shimeinheit 31 weist ebenfalls eine erste Trägerschublade 311, eine zweite Trägerschublade 312 und sechs weitere Trägerschubladen auf. Die Anzahl von jeweils acht Trägerschubladen ist dabei nur beispielhaft, üblicherweise sind 12 bis 24 oder noch mehr Trägerschubladen vorhanden. In die Trägerschubladen werden vor Inbetriebnahme der Magnetresonanzbildgebungseinheit nach Bedarf die entsprechende Anzahl von Shimelelementen eingelegt, um Magnetfeldverzerrungen, insbesondere solche höherer Ordnung (>1), zumindest teilweise, insbesondere um mindestens 50%/ 95%, zu kompensieren.

In der FIG 2 ist das medizinische Bildgebungssystem für den Fall gezeigt, dass der Hauptmagnet 20 um einen Rotationswinkel Θ rotiert ist. Die rotierbare passive Shimeinheit 21 dreht sich dabei ebenfalls um den Rotationswinkel Θ um das Drehzentrum (im Allgemeinen der Mittelpunkt der Patientenöffnung 40), während die statische passive Shimeinheit 31 sich nicht verändert. Das Koordinatensystem des statischen Bezugssystems (statischer Teil des Bildgebungssystems) hat dabei die Koordinaten x', y' und z'.

Die statische passive Shimeinheit 31 wird bei ihrer Bestückung mit Shimelementen derart bestückt, dass sie eine erste Komponente der Magnetfeldverzerrung, welche Komponente unabhängig von einem Rotationswinkel ist, zumindest teilweise kompensiert. Es kann vorgesehen sein, dass die statische passive Shimeinheit 31 die winkelunabhängige Komponente der Magnetfeldverzerrung zu mindestens 50%/ 95% kompensiert. Die statische passive Shimeinheit kompensiert also die Magnetfeldverzerrungskomponenten, welche durch die statischen Teile des Bildgebungssystems und durch die Umgebung hervorgerufen werden. Die rotierbare passive Shimeinheit 21 wird bei ihrer Bestückung mit Shimelementen derart bestückt, dass sie eine zweite Komponente der Magnetfeldverzerrung des Hauptmagneten, welche Komponente von einem Rotationswinkel abhängig ist, zumindest teilweise kompensiert. Es kann vorgesehen sein, dass die rotierbare passive Shimeinheit 21 die winkelabhängige Komponente der Magnetfeldverzerrung zu mindestens 95% kompensiert. Die rotierbare passive Shimeinheit kompensiert also die Magnetfeldverzerrungskomponenten, welche durch den rotierbaren Hauptmagneten und andere rotierbare Teile des Bildgebungssystems hervorgerufen werden.

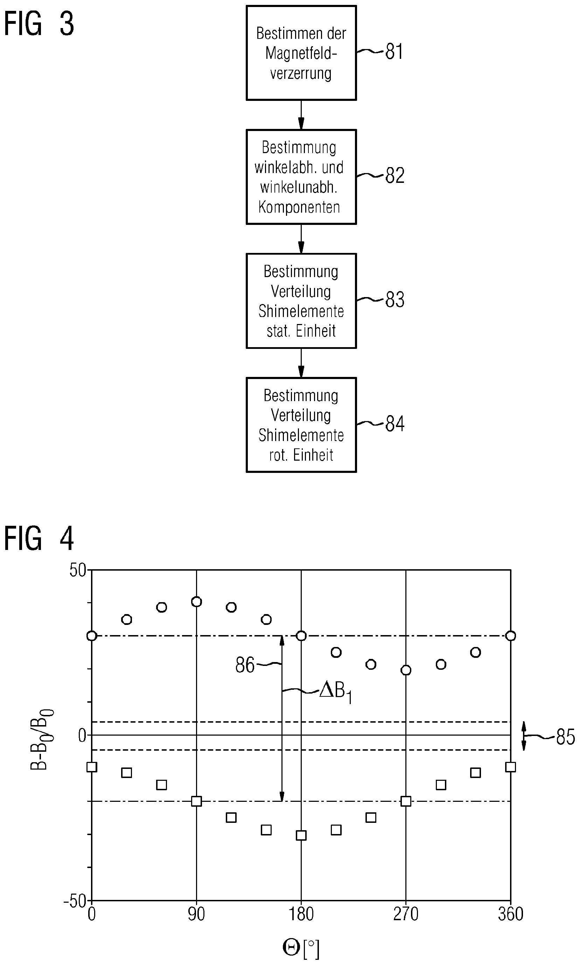

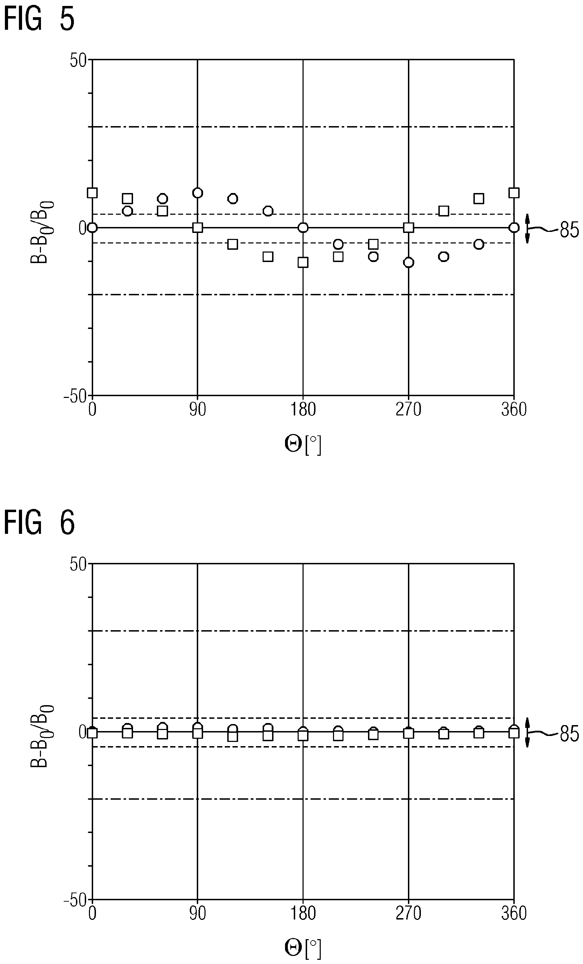

Dies kann z.B. anhand der Figuren 4 bis 6 gezeigt werden. In der FIG 4 ist die gesamte noch unkompensierte gemessene Magnetfeldverzerrung $\frac{B-B_0}{B_0}$ für zwei willkürlich gewählte Punkte innerhalb der Patientenöffnung gegenüber dem Rotationswinkel $\Theta$ (von 0° bis 360°) der rotierbaren Teile des Bildgebungssystems aufgetragen. B ist dabei das reale Magnetfeld und $B_0$ das angestrebte Magnetfeld ohne Magnetfeldverzerrung. Der angestrebte Schwellwert 85 der Magnetfeldverzerrung nach Kompensierung beträgt dabei z.B. 5% der ursprünglichen Magnetfeldverzerrung. Die winkelunabhängige erste Komponente 86 der Magnetfeldverzerrung ist durch einen Pfeil angedeutet. Wird die statische passive Shimeinheit 31 derart bestückt, dass die winkelunabhängige erste Komponente der Magnetfeldverzerrung (z.B. zu 95%) kompensiert wird, ist bei einer anschließenden Messung wie in FIG 5 gezeigt (ebenfalls gemessene Magnetfeldverzerrung $\frac{B-B_0}{B_0}$ für dieselben zwei Punkte wie in FIG 4 gegenüber dem Rotationswinkel $\Theta$ der rotierbaren Teile des Bildgebungssystems aufgetragen) nur noch die winkelabhängige Komponente der Magnetfeldverzerrung vorhanden. Wird diese anschließend durch die Bestückung der rotierbaren passiven Shimeinheit 21 kompensiert, so ergibt bei erneuter Messung der Magnetfeldverzerrung ein Bild wie in FIG 6 gezeigt, bei welcher der Rest der Magnetfeldverzerrung nur noch maximal den angestrebten Schwellwert 85 erreicht.

In der FIG 3 ist der Ablauf des erfindungsgemäßen Verfahrens zur Bestückung von Shim-Einheiten mit Shimelementen bei einem medizinischen Bildgebungssystem gezeigt. In einem ersten Schritt 81 wird die Feldverteilung $\Delta B(x', y', z', \Theta)$ des Magnetfeldes der Magnetresonanzbildgebungseinheit innerhalb der Patientenöffnung des Hauptmagneten abhängig von dem Rotationswinkel $\Theta$ gemessen und daraus die entsprechende Verteilung der Magnetfeldverzerrung bestimmt. Das sogenannte "Mappen" der Feldverteilung und Bestimmung der Magnetfeldverzerrung ist generell bekannt. Im vorliegenden Fall wird insbesondere die Winkelabhängigkeit beachtet, das heißt es werden die rotierbaren Teile der Magnetresonanzbildgebungseinheit rotiert und dabei für eine Vielzahl von Punkten innerhalb der Patientenöffnung bei einer Vielzahl von Rotationswinkeln Messungen durchgeführt. In einem zweiten Schritt 82 wird eine Aufteilung der Magnetfeldverzerrung in eine erste Komponente der Magnetfeldverzerrung, welche erste Komponente unabhängig vom Rotationswinkel ist und eine zweite Komponente, welche zweite Komponente abhängig vom Rotationswinkel ist, durchgeführt. Die Bestimmung der ersten und zweiten Komponente kann mittels einer Berechnungseinheit durch einen Algorithmus folgendermaßen berechnet werden:

$$\Delta B\left(x^{'},y^{'},z^{'},\Theta\right) = \Delta B_1\left(x^{'},y^{'},z^{'}\right) + \Delta B_2\left(x^{'},y^{'},z^{'},\Theta\right)$$

$B_1$ ist dabei die erste, rotationswinkelunabhängige Komponente und $B_2$ die zweite rotationswinkelabhängige Komponente der Magnetfeldverzerrung. X', y' und z' sind dabei die Ortkoordinaten im nicht-rotierenden Bezugssystem, also in dem Teil des Bildgebungssystems, welches statisch ist.

Der dritte Schritt 83 und der vierte Schritt 84 können auch in abgeänderter Reihenfolge durchgeführt werden. In einem dritten Schritt 83 wird die Verteilung der Shimelemente in der statischen Shim-Einheit 31 zur Kompensierung der ersten Komponente der Magnetfeldverzerrung bestimmt bzw. berechnet, also welche Shimelemente in welche Trägerschubladen der statischen Shim-Einheit eingelegt werden müssen, um eine Kompensierung der ersten Komponente der Magnetfeldverzerrung zu erzielen. In einem vierten Schritt 84 wird die Verteilung der Shimelemente der rotierbaren Shim-Einheit 21 zur Kompensierung der zweiten (winkelabhängigen) Komponente der Magnetfeldverzerrung bestimmt bzw. berechnet, also welche Shimelemente in welche Trägerschubladen der rotierbaren Shim-Einheit eingelegt werden müssen, um eine Kompensierung der zweiten Komponente der Magnetfeldverzerrung zu erzielen. Insbesondere wird die Anzahl, Größe und Position der Shimelemente in den Shim-Einheiten bestimmt bzw. berechnet, um anschließend die Bestückung auf einfache Weise durchführen zu können.

Nach einer weiteren Ausgestaltung der Erfindung werden die Shimelemente gemäß der berechneten Verteilung in die Shim-Einheiten eingelegt. Dies kann manuell z.B. durch einen Servicetechniker oder auch automatisch durch eine Bestückungsvorrichtung durchgeführt werden. Anschließend kann durch nochmaliges Messen der Feldverteilung des Magnetfeldes überprüft werden, ob die Kompensierung ausreichend ist, eventuell kann eine weitere oder iterativ mehrere Berechnung(en) und Bestückung(en) durchgeführt werden. Alternativ kann auch nach dem dritten Schritt 83 bereits eine Bestückung der entsprechenden Shim-Einheit und Nachmessen der Magnetfeldverzerrung stattfinden und erst dann die Berechnung der Bestückung der zweiten passiven Shim-Einheit durchgeführt werden.

Für den Fall, dass ein Bildgebungssystem einen statischen Hauptmagneten und eine rotierbare Gantry mit einer Röntgenbildgebungseinheit aufweist, so rotiert die rotierbare passive Shim-Einheit mit der Gantry um den Hauptmagneten, während die statische passive Shim-Einheit fest bleibt. Auch hier erfolgt in gleicher Weise wie oben beschrieben eine Aufteilung der zu kompensierenden Komponenten der Magnetfeldverzerrung in rotationswinkelabhängige und rotationswinkelunabhängige Komponenten.

Alternativ kann statt einer Röntgenbildgebungseinheit auch eine andere Strahlungserzeugungseinheit, z.B. ein LINAC vorgesehen sein.

Zusätzlich zu den passiven Shimeinheiten 21, 31 kann auch eine oder mehrere aktive Shimeinheiten (nicht gezeigt) vorgesehen sein. Eine aktive Shimeinheit weist im Allgemeinen ansteuerbare Spulen auf, welche flexibel jederzeit derart geschaltet werden können, dass sie entsprechende Magnetfeldverzerrungskomponenten, die noch unkompensiert sind, kompensieren. Durch die aktive Shimeinheit wird z.B. die Rest-Magnetfeldverzerrung (z.B. 5%) oder eine zeitlich schwankende Magnetfeldverzerrungskomponente oder die Magnetfeldverzerrung nullter bzw. erster Ordnung kompensiert. Die aktive Shimeinheit kann z.B. winkelabhängig angesteuert werden.

Um die Feldhomogenität des Magnetfelds auch in Bezug auf Verzerrungen höherer Ordnung und in Bezug auf alle Rotationswinkel zu verbessern ist folgendes vorgesehen: Medizinisches Bildgebungssystem zur kombinierten Magnetresonanzbildgebung und Bestrahlung eines Untersuchungsobjektes, aufweisend eine Magnetresonanzbildgebungseinheit und eine Strahlungserzeugungseinheit, welche mechanisch miteinander derart verbunden sind, dass die Strahlungserzeugungseinheit mit der Magnetresonanzbildgebungseinheit baulich integriert ist und beide eine Patientenöffnung zur Lagerung des Untersuchungsobjektes umgeben, wobei die Magnetresonanzbildgebungseinheit zumindest einen Hauptmagneten zur Erzeugung eines Magnetfelds aufweist und wobei die Strahlungserzeugungseinheit eine Strahlenquelle zur Erzeugung einer Strahlung aufweist, wobei das Bildgebungssystem aus einem statischen Teil und einem um einen Rotationswinkel rotierbaren Teil derart ausgebildet ist, dass entweder zumindest ein drehbarer Hauptmagnet um eine statische Strahlungserzeugungseinheit rotierbar angeordnet ist oder eine drehbare Strahlungserzeugungseinheit um zumindest einen statischen Hauptmagneten rotierbar angeordnet ist, wobei das Bildgebungssystem zumindest zwei passive Shim-Einheiten aufweist, von denen zumindest eine statische Shim-Einheit feststehend ist und zumindest eine drehbare Shim-Einheit mit dem rotierbaren Teil fest verbunden ist

## Patentansprüche

1. Medizinisches Bildgebungssystem zur kombinierten Magnetresonanzbildgebung und Bestrahlung eines Untersuchungsobjektes, aufweisend eine Magnetresonanzbildgebungseinheit und eine Strahlungserzeugungseinheit, welche mechanisch miteinander derart verbunden sind, dass die Strahlungserzeugungseinheit mit der Magnetresonanzbildgebungseinheit baulich integriert ist und beide eine Patientenöffnung zur Lagerung des Untersuchungsobjektes umgeben, wobei die Magnetresonanzbildgebungseinheit zumindest einen Hauptmagneten zur Erzeugung eines Magnetfelds aufweist und wobei die Strahlungserzeugungseinheit eine Strahlenquelle zur Erzeugung einer Strahlung aufweist, wobei das Bildgebungssystem aus einem statischen Teil und einem um einen Rotationswinkel rotierbaren Teil derart ausgebildet ist, dass entweder zumindest ein drehbarer Hauptmagnet um eine statische Strahlungserzeugungseinheit rotierbar angeordnet ist oder eine drehbare Strahlungserzeugungseinheit um zumindest einen statischen Hauptmagneten rotierbar angeordnet ist, wobei das Bildgebungssystem zumindest zwei passive Shim-Einheiten aufweist, von denen zumindest eine statische Shim-Einheit feststehend ist und zumindest eine drehbare Shim-Einheit mit dem rotierbaren Teil fest verbunden ist.

2. Medizinisches Bildgebungsgerät nach Anspruch 1, wobei die zumindest zwei passiven Shim-Einheiten jeweils eine Vielzahl von Shimelementen aufweisen und wobei die Shimelemente in den Shim-Einheiten derart angeordnet sind, dass sie Magnetfeldverzerrungen zumindest teilweise, insbesondere zu mindestens 95%, kompensieren.

3. Medizinisches Bildgebungsgerät nach Anspruch 2, wobei die zu kompensierenden Magnetfeldverzerrungen zumindest zweiter und/oder höherer Ordnung sind.

4. Medizinisches Bildgebungsgerät nach einem der vorangehenden Ansprüche, wobei die zumindest eine statische Shim-Einheit derart ausgebildet ist, dass sie eine erste Komponente der Magnetfeldverzerrungen, welche unabhängig von dem Rotationswinkel ist, zumindest teilweise, insbesondere zu mindestens 95%, kompensiert und die zumindest eine drehbare Shim-Einheit eine zweite Komponente der Magnetfeldverzerrungen, die abhängig von dem Rotationswinkel ist, zumindest teilweise, insbesondere zu mindestens 95%, kompensiert.

5. Medizinisches Bildgebungsgerät nach einem der vorangehenden Ansprüche, wobei die Strahlungserzeugungseinheit von einer Röntgenbildgebungseinheit gebildet wird, und die Röntgenbildgebungseinheit eine Röntgenquelle zur Erzeugung eines Röntgenstrahls aufweist.

6. Medizinisches Bildgebungsgerät nach Anspruch 5, wobei die Röntgenbildgebungseinheit außerdem einen Röntgendetektor zur Umwandlung von Strahlung in Bilddaten aufweist.

7. Medizinisches Bildgebungsgerät nach einem der vorangehenden Ansprüche, zusätzlich aufweisend zumindest eine aktive Shimeinheit mit einer Vielzahl von Spulen, wobei die Spulen bezüglich ihres Durchflussstroms ansteuerbar ausgebildet sind.

8. Verfahren zur Bestimmung der Bestückung von Shim-Einheiten mit Shimelementen bei einem medizinischen Bildgebungssystem nach einem der Ansprüche 1 bis 7, mit den folgenden Schritten:

   • Messen der Feldverteilung des Magnetfeldes der Magnetresonanzbildgebungseinheit, insbesondere innerhalb der Patientenöffnung, abhängig von dem Rotationswinkel und daraus Bestimmung der Magnetfeldverzerrung,
   • Bestimmung einer ersten Komponente der Magnetfeldverzerrung, die unabhängig vom Rotationswinkel ist und einer zweite Komponente, die abhängig vom Rotationswinkel ist,
   • Bestimmung der Verteilung von Shimelementen in der statischen Shim-Einheit zur Kompensierung der ersten Komponente, und
   • Bestimmung der Verteilung von Shimelementen in der drehbaren Shim-Einheit zur Kompensierung der zweiten Komponente.

9. Verfahren nach Anspruch 8, wobei die Anzahl, Größe und Position der Shimelemente in den Shim-Einheiten bestimmt oder berechnet wird.

10. Verfahren nach Anspruch 8 oder 9, wobei die Shimelemente gemäß der berechneten Verteilung in die Shim-Einheiten eingelegt werden.

# FIG 1

# FIG 2

FIG 3

```
┌─────────────────┐
│  Bestimmen der  │
│   Magnetfeld-   │──~81
│   verzerrung    │
└─────────────────┘
         │
         ▼
┌─────────────────┐
│   Bestimmung    │
│  winkelabh. und │──~82
│  winkelunabh.   │
│   Komponenten   │
└─────────────────┘
         │
         ▼
┌─────────────────┐
│   Bestimmung    │
│   Verteilung    │──~83
│  Shimelemente   │
│   stat. Einheit │
└─────────────────┘
         │
         ▼
┌─────────────────┐
│   Bestimmung    │
│   Verteilung    │──~84
│  Shimelemente   │
│   rot. Einheit  │
└─────────────────┘
```

FIG 4

## FIG 5

## FIG 6

**Europäisches Patentamt**
**European Patent Office**
**Office européen des brevets**

# EUROPÄISCHER RECHERCHENBERICHT

Nummer der Anmeldung

EP 17 19 3198

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | US 2017/014644 A1 (SHVARTSMAN SHMARYU M [US] ET AL) 19. Januar 2017 (2017-01-19) | 1-4,7 | INV. G01R33/3873 G01R33/48 |
| Y | * Absätze [0012], [0045], [0052], [0062] - [0064]; Abbildungen 1A, 1B * ----- | 5,6,8-10 | |
| Y,D | SJOERD CRIJNS ET AL: "From static to dynamic 1.5T MRI-linac prototype: impact of gantry position related magnetic field variation on image fidelity", PHYSICS IN MEDICINE AND BIOLOGY, INSTITUTE OF PHYSICS PUBLISHING, BRISTOL GB, Bd. 59, Nr. 13, 29. Mai 2014 (2014-05-29), Seiten 3241-3247, XP020266457, ISSN: 0031-9155, DOI: 10.1088/0031-9155/59/13/3241 [gefunden am 2014-05-29] * section 2.2 * ----- | 8-10 | ADD. G01R33/3875 |
| Y,D | WACHOWICZ K ET AL: "Geometric distortion and shimming considerations in a rotating MR-linac design due to the influence of low-level external magnetic fields", MEDICAL PHYSICS, AIP, MELVILLE, NY, US, Bd. 39, Nr. 5, 19. April 2012 (2012-04-19), Seiten 2659-2668, XP012161022, ISSN: 0094-2405, DOI: 10.1118/1.3702591 [gefunden am 2012-04-19] * chapter II.C. * ----- -/-- | 8-10 | RECHERCHIERTE SACHGEBIETE (IPC) G01R |

Der vorliegende Recherchenbericht wurde für alle Patentansprüche erstellt

| Recherchenort | Abschlußdatum der Recherche | Prüfer |
|---|---|---|
| München | 2. März 2018 | Skalla, Jörg |

Seite 1 von 2

**Europäisches Patentamt**
**European Patent Office**
**Office européen des brevets**

# EUROPÄISCHER RECHERCHENBERICHT

Nummer der Anmeldung

EP 17 19 3198

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | WANG GE ET AL: "Vision 20/20: Simultaneous CT-MRI - Next chapter of multimodality ima", MEDICAL PHYSICS, AIP, MELVILLE, NY, US, Bd. 42, Nr. 10, 17. September 2015 (2015-09-17), Seiten 5879-5889, XP012200566, ISSN: 0094-2405, DOI: 10.1118/1.4929559 [gefunden am 1901-01-01] * chapter 3.B. * ----- | 5,6 | |
| A | US 2016/144200 A1 (LEACH JEFFREY EDWARD [US] ET AL) 26. Mai 2016 (2016-05-26) * Absätze [0026], [0034] * ----- | 1 | |
| A | WEN ZHIFEI ET AL: "Shimming with permanent magnets for the x-ray detector in a hybrid x-ray/MR system", MEDICAL PHYSICS, AIP, MELVILLE, NY, US, Bd. 35, Nr. 9, 7. August 2008 (2008-08-07), Seiten 3895-3902, XP012116221, ISSN: 0094-2405, DOI: 10.1118/1.2963994 * sections II, II.A.1; Abbildung 1 * ----- | 1 | RECHERCHIERTE SACHGEBIETE (IPC) |

Der vorliegende Recherchenbericht wurde für alle Patentansprüche erstellt

| Recherchenort | Abschlußdatum der Recherche | Prüfer |
|---|---|---|
| München | 2. März 2018 | Skalla, Jörg |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
..............................................................
& : Mitglied der gleichen Patentfamilie, übereinstimmendes Dokument

EPO FORM 1503 03.82 (P04C03)

## ANHANG ZUM EUROPÄISCHEN RECHERCHENBERICHT
## ÜBER DIE EUROPÄISCHE PATENTANMELDUNG NR.

EP 17 19 3198

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

02-03-2018

| Im Recherchenbericht angeführtes Patentdokument | Datum der Veröffentlichung | Mitglied(er) der Patentfamilie | Datum der Veröffentlichung |
|---|---|---|---|
| US 2017014644 A1 | 19-01-2017 | AU 2010273298 A1 | 10-11-2011 |
| | | AU 2015200287 A1 | 12-02-2015 |
| | | AU 2017200361 A1 | 09-02-2017 |
| | | CA 2760055 A1 | 20-01-2011 |
| | | CN 102472830 A | 23-05-2012 |
| | | CN 105664378 A | 15-06-2016 |
| | | EP 2454617 A1 | 23-05-2012 |
| | | JP 5719844 B2 | 20-05-2015 |
| | | JP 6224020 B2 | 01-11-2017 |
| | | JP 2012533362 A | 27-12-2012 |
| | | JP 2015134211 A | 27-07-2015 |
| | | JP 2017221862 A | 21-12-2017 |
| | | US 2011012593 A1 | 20-01-2011 |
| | | US 2015065860 A1 | 05-03-2015 |
| | | US 2017014644 A1 | 19-01-2017 |
| | | WO 2011008969 A1 | 20-01-2011 |
| US 2016144200 A1 | 26-05-2016 | CN 105324678 A | 10-02-2016 |
| | | EP 3011354 A1 | 27-04-2016 |
| | | JP 2016526968 A | 08-09-2016 |
| | | RU 2016101577 A | 26-07-2017 |
| | | US 2016144200 A1 | 26-05-2016 |
| | | WO 2014203190 A1 | 24-12-2014 |

EPO FORM P0461

Für nähere Einzelheiten zu diesem Anhang : siehe Amtsblatt des Europäischen Patentamts, Nr.12/82

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Nicht-Patentliteratur**

- **SJOERD CRIJNS ; BAS RAAYMAKERS.** From static to dynamic 1.5T MRI-linac prototype: impact of gantry position related magnetic field variation on image fidelity. *Phys. Med. Biol.,* 2014, vol. 59, 3241-3247

- **K. WACHOWICZ ; T. TADIC ; B.G. FALLONE.** Geometric distortion and shimming considerations in a rotating MR-linac design due to the influence of low-level externa magnetic fields. *Med. Phys.,* 2012, vol. 39 (5), 2659-2668